# EUROPEAN PATENT APPLICATION

(11) **EP 4 067 479 A1**
(43) Date of publication of application: **05.10.2022**
(21) Application number: 20894710.1
(22) Date of filing: 22.10.2020
(51) Int. Cl.: C12N 5/04, C12P 17/06

(54) **METHOD FOR INCREASING THE CONTENT OF FLAVONOID PHENYLPROPANOID COMPOUNDS IN SAUSSUREA INVOLUCRATA CELL CULTURE**

(30) Priority: 28.11.2019 CN 201911194580
(71) Applicant: Dalian Practical Biotechnology Co., Ltd., Dalian, Liaoning 116000 (CN)
(72) Inventor: LIU, Hanshi, Dalian, Liaoning 116000 (CN); JIN, Yinbing, Dalian, Liaoning 116000 (CN); LIU, Yaping, Dalian, Liaoning 116000 (CN)
(74) Representative: Proi World Intellectual Property GmbH
(86) International application number: PCT/CN2020/122753
(87) International publication number: WO 2021/103863

(57) **Abstract**

The present invention belongs to the field of plant biotechnology engineering, and particularly relates to a method for improving flavonoid phenylpropanoid compounds in a *Saussurea involucrata* cell culture. In a medium used in the method, a phosphorus concentration is 0.5-3 mmol/L, a NO₃⁻ ion concentration is 20-35 mmol/L, a NH₄⁺ ion concentration is 0-30 mmol/L, a Ca²⁺ ion concentration is 0.5-3 mmol/L, a Mg²⁺ ion concentration is 0.2-1.5 mmol/L, and a boron ion concentration is 0.02-0.1 mmol/L; an inducer or precursor or bypass metabolism inhibitor may be added to the medium. Finally, the content of total flavonoids and the contents of rutin, chlorogenic acid, syringin and 1,5-dicaffeoylquinic acid in the *Saussurea involucrata* cell culture are significantly improved.

## Description

### TECHNICAL FIELD

The present invention belongs to the field of plant biotechnology engineering, and relates to a tissue culture of a medicinal plant and a culture method thereof, in particular to a method for improving flavonoid phenylpropanoid compounds in a *Saussurea involucrata* cell culture.

### BACKGROUND

*Saussurea involucrata (Kar. et Kir.) Sch.-Bip.* (*Saussurea involucrata* for short), a folk medicinal plant in alpine areas, is used for dispelling cold and removing dampness, activating blood to promote menstruation, resisting inflammation and easing pain, and mainly used in treatment of symptoms such as rheumarthritis, coldness and pain in the lower abdomen of women, amenorrhea, retained afterbirth, paralysis, cough with syndrome of cold invading lung, impotence and mountain sickness. In recent years, the effects of *Saussurea involucrata* as an ethnic drug have attracted much attention in resistance to inflammation, pain alleviation, anti-early pregnancy, anti-aging and inhibition of cancer cell proliferation. However, *Saussurea involucrata* plants are mainly distributed in cold plateau regions above 4,000 m, such as Xinjiang, Gansu, Sichuan, Yunnan and Tibet. In these regions, climates are changeable, and weathers are unpredictable. The maximum monthly average temperature is 3-10°C, while the minimum monthly average temperature ranges from more than 10 degrees below zero to dozens of degrees below zero. The growing environment is very bad, and there are only hardy carex, kobresia and a few alpine perennial herbs growing together with *Saussurea involucrata.* Generally, the *Saussurea involucrata* plants in such environments are mostly perennial and grow slowly, and artificial cultivation is difficult. For a long time, predatory excavation has resulted in a serious shortage of *Saussurea involucrata* resources, and *Saussurea involucrata* has become an endangered species. It is difficult for natural resources to satisfy the ever-increasing clinical needs.

Flavonoids, flavonoid glycosides, sesquiterpene lactones, coumarins, lignans, steroids, polysaccharides and other components contained in *Saussurea involucrata* have biological activities in anti-rheumatism, pain alleviation, regulation of cardiovascular system, anti-cancer, anti-aging, family planning, radiation protection and the like. Thus, *Saussurea involucrata* has a great development and utilization value and a good application prospect. Studies have proved that: syringin has anti-inflammatory, analgesic, anti-hyperglycemic, anti-depression and anti-cancer activities; chlorogenic acid has anti-inflammatory, anti-hyperglycemic and anti-hepatitis B virus effects; 1,5-dicaffeoylquinic acid has the effect of antioxidant therapy for diabetes and the function of anti-AIDS and has entered the stage of clinical research as a new anti-AIDS drug. To sum up, the syringin, chlorogenic acid and 1,5-dicaffeoylquinic acid have a variety of medicinal activities and important application values. The syringin, chlorogenic acid and 1,5-dicaffeoylquinic acid are active components of wild *Saussurea involucrata,* but their contents are low. It is promising to produce the three components by the *Saussurea involucrata* cell culture.

Development of *Saussurea involucrata* by applying the cell culturing methods can not only satisfy social demands for *Saussurea involucrata* resources, but also protect natural resources and maintain ecological environments. However, in research reports on tissue culturing of *Saussurea involucrata,* the application of *Saussurea involucrata* in actual production is greatly limited due to many problems as follows:
1. The obtained culture has simple components, single total flavonoids mostly serve as the index, the content of the total flavonoids is low, so it is difficult to realize a real value of *Saussurea involucrata;* 2. No effective means of obtaining a high-yield cell line has been found to solve the problem of low contents of some components; 3. An inducer of the culture is only used to obtain a higher content of total flavonoids, but other components of *Saussurea involucrata* itself are ignored; 4. Addition of the inducer is emphasized only in the process of metabolic regulation, and there is no method for blocking the synthesis of unwanted metabolites by changing a flow direction of a branched metabolic pathway to improve the product yield; 5. Common minimal media are mostly used in the culturing process, but no minimal medium specific to *Saussurea involucrata* has been established according to the features of *Saussurea involucrata* and the quality of the culture; and 6. most cultures remain in a laboratory, large-scale culturing is difficult to realize, the cultures are unstable in the process of scale-up culturing, and the contents of active components will be reduced after the scale-up culturing reaches a certain scale. To sum up, researchers at home and abroad have done a lot of work in callus culturing, cell suspension culturing, fermentation culturing and the like of *Saussurea involucrata.* However, due to simple culturing effects of *Saussurea involucrata* cells and simple medicinal components of *Saussurea involucrata* cultures, the improvement in the contents of main medicinal components needs to be explored. Due to no optimization of basic culture environments, less regulation of the metabolic pathway and the like, there is no stable, high-yield and fast regulation process for large-scale culturing of *Saussurea involucrata* cells.

### SUMMARY

In order to overcome the defects of the prior art, the present invention provides a method for improving flavonoid phenylpropanoid compounds in a *Saussurea involucrate* cell culture.

In order to achieve the above-mentioned purpose, the following technical solution is adopted in the present invention:
A medium for improving flavonoid phenylpropanoid compounds in a *Saussurea involucrata* cell culture is provided; in the medium, a phosphorus concentration is 0.5-3 mmol/L, a NO₃⁻ ion concentration is 20-35 mmol/L, a NH₄⁺ ion concentration is 0-30 mmol/L, a Ca²⁺ ion concentration is 0.5-3 mmol/L, a Mg²⁺ ion concentration is 0.2-1.5 mmol/L, and a boron ion concentration is 0.02-0.1 mmol/L.

Further, in the medium, a phosphorus concentration is 2.7 mmol/L, a NO₃⁻ ion concentration is 30 mmol/L, a NH₄⁺ ion concentration is 18 mmol/L, a Ca²⁺ ion concentration is 0.6 mmol/L, a Mg²⁺ ion concentration is 0.3 mmol/L, and a boron ion concentration is 0.03 mmol/L.

Further, the medium includes potassium nitrate 3000 mg/L, ammonium sulfate 793 mg/L, diammonium hydrogen phosphate 340 mg/L, magnesium sulfate heptahydrate 74 mg/L, calcium chloride dihydrate 88 mg/L, potassium iodide 0.83 mg/L, boric acid 2 mg/L, manganese sulfate tetrahydrate 22.3 mg/L, ferrous sulfate heptahydrate 27.8 mg/L, zinc sulfate heptahydrate 8.6 mg/L, inositol 100 mg/L, sodium molybdate dihydrate 0.25 mg/L, thiamine hydrochloride 0.1 mg/L, copper sulfate pentahydrate 0.025 mg/L, pyridoxine hydrochloride 0.5 mg/L, cobalt chloride hexahydrate 0.025 mg/L, nicotinic acid 0.5 mg/L, ethylene diamine tetraacetic acid disodium 37.3 mg/L, glycine 2 mg/L.

An inducer for improving flavonoid phenylpropanoid compounds in a *Saussurea involucrate* cell culture is provided; the inducer is one or more of SNP inducer, MeJ inducer, SA inducer, and silver ion and silver ion complex inducer; wherein a concentration of the SNP inducer added is 1-50 mmol/L, a concentration of the MeJ inducer added is 1-50 mg/L, a concentration of the SA inducer added is 1-50 mg/L, and a concentration of the silver ion and silver ion complex inducer added is 1-50 mg/L.

Further, the concentration of the SNP inducer added is 10 mmol/L, the concentration of the MeJ inducer added is 20 mg/L, the concentration of the SA inducer added is 30 mg/L, and the concentration of the silver ion and silver ion complex inducer added is 20 mg/L.

A precursor for improving flavonoid phenylpropanoid compounds in a *Saussurea involucrate* cell culture is provided; the precursor is one or more of cinnamic acid, phenylalanine, sodium acetate, tyrosine and oxalic acid; wherein a concentration of the cinnamic acid added is 1-20 mg/L, a concentration of the phenylalanine added is 1-20 mg/L, a concentration of the sodium acetate added is 1-20 mg/L, a concentration of the tyrosine added is 1-50 mg/L, and a concentration of the oxalic acid added is 1-20 mg/L.

Further, the concentration of the cinnamic acid added is 10 mg/L, the concentration of the phenylalanine added is 10 mg/L, the concentration of the sodium acetate added is 10 mg/L, the concentration of the tyrosine added is 20 mg/L, and the concentration of the oxalic acid added is 10 mg/L.

A bypass metabolism inhibitor for improving synthesis of flavonoid phenylpropanoid compounds in a *Saussurea involucrate* cell culture is provided; the bypass metabolism inhibitor is one or more of arginine, trisodium citrate, sodium fluoride, cyclohexylalanine and 5-MT (5-methoxytryptophan); wherein a concentration of the arginine is 0-100 mg/L, a concentration of the trisodium citrate is 0-50 mg/L, a concentration of the sodium fluoride is 0-100 mg/L, a concentration of the cyclohexylalanine is 0-100 mg/L, and a concentration of the 5-MT (5-methoxytryptophan) is 0-100 mg/L.

Further, the concentration of the arginine added is 10 mg/L, the concentration of the trisodium citrate added is 15 mg/L, the concentration of the sodium fluoride added is 15 mg/L, the concentration of the cyclohexylalanine added is 10 mg/L, and the concentration of the 5-MT (5-methoxytryptophan) added is 10 mg/L.

A method for improving flavonoid phenylpropanoid compounds in a *Saussurea involucrate* cell culture is provided, wherein the method includes the following steps:
(1) acquisition of a primitive cell line:
   using a seed embryo of *Saussurea involucrata* or a plant germinated from a seed as an explant, inducing a callus with an induction medium, and performing repeated subculturing to obtain a *Saussurea involucrate* cell line;
(2) inoculating the *Saussurea involucrata* cell line obtained in step (1) into a medium for culturing, with an inoculation amount of 1-50 g of cells (fresh weight) per liter of medium, an illumination amount of 1,000-3,000 lxu, an illumination time of 12-24 h/day and a shaker speed of 100-130 rpm, performing subculturing one time every 7 days-20 days, and establishing a stable and high-yield *Saussurea involucrata* suspension cell culture line after 3-4 rounds of subculturing; and
(3) large-scale culturing: selecting the culture in step (2), inoculating the culture into a medium for culturing, with an inoculation amount of 10-80 g of cells (fresh weight) per liter of medium, and filtering and harvesting cells after suspension culturing for 14-20 days;
   wherein the medium in step (2) and step (3) is the aforementioned medium; or the medium is a medium including the aforementioned precursor; or the medium is a medium including the aforementioned inducer; or the medium is a medium including the aforementioned inhibitor for inhibiting bypass metabolism.

Further, when the precursor is cinnamic acid, phenylalanine or sodium acetate, the precursor is added during inoculation; when the precursor is tyrosine or oxalic acid, the precursor is added in the middle stage of culturing; the inducer is added in the middle stage of culturing; and the bypass metabolism inhibitor is added in the late stage of culturing.

Further, before the large-scale culturing in step (3), the cells are inoculated into culture flasks containing 5-20 glass beads with a diameter of 1-10 mm, the culturing conditions are the same as those of the primitive cells, the culture flasks with *Saussurea involucrata* plants not growing well are discarded in the subculturing process, and a shear-resistant, stable and high-yield *Saussurea involucrata* suspension cell culture line is established after 10 rounds.

**Beneficial effects** of the present invention: According to the method of the present invention, the medium, inducer, precursor and bypass metabolism inhibitor of the present invention are used in the process of preparing a *Saussurea involucrate* cell culture, so that the content of total flavonoids and the contents of rutin, chlorogenic acid, syringin and 1,5-dicaffeoylquinic acid in the *Saussurea involucrata* cell culture are significantly increased.

### DETAILED DESCRIPTION OF THE EMBODIMENTS

The present invention will be further described below in conjunction with the specific embodiments, but such embodiments should not limit the present invention in any way.

### Example 1 Cultivation of Saussurea involucrata sterile seedling

Natural *Saussurea involucrata* seeds that had been identified by the Herbarium of Institute of Botany, the Chinese Academy of Sciences (National Herbarium) were taken, soaked in 75% alcohol for 10-30 s at first, then disinfected with 1% mercuric chloride solution for 8-12 min, repeatedly washed with sterile water 4-5 times, finally placed on sterile filter paper which absorbed surface moisture of the seeds, inoculated by sterile forceps onto a solid medium obtained by adding 0.5-3 mg/L GA (gibberellin), 10-30 g/L saccharose and 0.6 g/L agar into 1/2MS (a formula as shown in Table 3), and cultured under light at 22-27 °C. The seeds germinated in about 10 days, and sterile seedlings sprouted out.

### Example 2 Induction and culturing of callus

Roots, stems, leaves, cotyledons and germs of sterile seedlings were taken as explants, cut into 2-5 mm long small pieces under sterile conditions by sterile surgical scissors and sterile forceps, inoculated onto a solid medium obtained by adding 0.2 mg/L-0.5 mg/L 6-BA (6-benzyladenine), 2 mg/L-4 mg/L NAA (naphthalene acetic acid), 0.6 g/L agar and 30 g/L saccharose into MS (a formula as shown in Table 2), cultured in the dark at 22-27°C, and a callus was induced in 7-10 days.

### Example 3 Subculturing and screening of callus

Four *Saussurea involucrata* cell lines (i.e., white, yellow, green and purplish red cell lines) were obtained through induction and culturing of the *Saussurea involucrata* callus; and the four cell lines were subcultured respectively. By testing a growth speed and a content of total flavonoids of the four cell lines, it was determined that the purplish red cell line grew well and had the highest content of total flavonoids.

The purplish red cell line was selected as a primitive cell line, a dark and bright purplish red callus was selected from the solid medium by a visual observation method, and subcultured on a solid medium obtained by adding 0.2 mg/L-0.5 mg/L 6-BA (6-benzyladenine), 2 mg/L-4 mg/L NAA (naphthalene acetic acid), 0.6 g/L agar and 30 g/L saccharose were added into MS (a formula as shown in Table 2). The size of an inoculation block was controlled to be less than 3 mm for subculturing in order to optimize seeds and purify cell lines in a better way. These steps were repeated in this way, the selected cell line was domesticated progressively, and after nearly 100 rounds of screening, a stable and high-yield *Saussurea involucrata* cell line that could be subcultured indefinitely was obtained.

### Example 4 Establishment of a seed culture line based on liquid suspension culturing

The amplified *Saussurea involucrata* cells of example 3 were inoculated into a liquid synthetic medium (as shown in Table 1) + 0.2 mg/L-0.5 mg/L 6-BA (6-benzyladenine), 2 mg/L-4 mg/L NAA (naphthalene acetic acid) and 30 g/L saccharose for shake-flask suspension culturing, specifically: a culture flask was used as a culture vessel, a shaker was used for rotating and shaking culturing, an inoculation amount was 1-50 g of cells (fresh weight) per liter of medium, a temperature was 25±2°C, an illumination amount was 1,000-3,000 lxu, an illumination time was 12-24 h/day and a shaker speed was 100-130 rpm, subculturing was performed one time every 7 days-20 days, and a stable and high-yield *Saussurea involucrata* suspension cell culture line was established after 3-4 rounds of subculturing.

### Example 5 Establishment of seeds for a stirred tank fermenter

The *Saussurea involucrata* cells in example 3 or 4 were inoculated into culture flasks containing 5-20 glass beads with a diameter of 1-10 mm, the medium was the same as that used for the primitive cells, and the culturing conditions were as follows: 25±2°C, an illumination amount of 1,000-3,000 lxu, an illumination time of 12-24 h/day and a shaker speed of 100-130 rpm. Subculturing was performed one time every 7 days-20 days, the culture flasks with *Saussurea involucrate* plants not growing well were discarded in the subculturing process, and a shear-resistant, stable and high-yield *Saussurea involucrata* suspension cell culture line was established after 10 rounds. Subculturing and optimization were continuously performed on the optimized cell line in the culture flasks with the same shearing force, and the cultures were used for scale-up culturing in a fermenter in a next step.

### Example 6 Large-scale culturing of Saussurea involucrata cells

1.1 Seed selection
   Cultures cultured for 8-12 days with good growth trend, bright purplish red color, uniform particles, homogeneous texture and easy separation were selected from the cultures in example 3 and used as seeds.
1.2 Preparation of medium
   1,000 ml culture flasks were used, each flask was filled with 500 ml of culture solution, and the culture solution was prepared by adding 0.4 mg/L 6-BA (6-benzyladenine), 2 mg/L NAA (naphthalene acetic acid) and 30 g/L saccharose to a MS medium (as shown in Table 2) as a base, and the pH value was 5.5; high temperature sterilization: 121°C, 20 min.
1.3 Inoculation
   The cultures were inoculated into culture flasks filled with culture solution under sterile conditions, with an inoculation amount of 5% (W/V, g.fw/ml).
1.4 Shaker culturing
   Culturing conditions: the culturing temperature was 25 ± 2 °C, the shaker rotating speed was 110 rpm, the culturing time was 9 d, and the illumination conditions were 11 h/day and 2,000 lux.
   The cultures were frequently observed in the culturing process. If contamination was detected, the affected culture was picked out immediately.
3 Collection and recycling production of cultures
3.1 Reservation of seeds
   Calluses having the characteristics of vigorous growth, bright and purplish red color, compact structure, granular shape, homogeneous texture and slight hardness were selected during large-scale production to reserve seeds, and a suitable amount of seeds was no more than 15% of gross yield.
3.2 Collection of cultures

Residual cultures were collected and subjected to low-temperature vacuum drying. Dry cultures were ground into coarse or fine powder or used to extract flavonoids and other active components as required.

According to measurements, the large-scale production carried out by this method was low in cost and fast in culture growth rate, and the yield was up to 20-25 g (dry weight)/month/liter of medium; the content of total flavonoids in dry cultures was high, up to 8-12% of the dry weight of the cultures. According to the results of HPLC detection, the content of rutin in the cells was 0.0003%, the content of 1.5-O-dicaffeoylquinic acid was 0.8%, the content of syringin was 0.31%, and the content of chlorogenic acid was 0.35%.

### Example 7 Large-scale production of high-content cell culture using a synthetic medium

Seeds from the amplified high-yield *Saussurea involucrata* suspension cell culture line, which were cultured for 8-12 days with good growing trend, bright purplish red color, uniform particles and homogeneous texture, were selected from the cultures in example 4. Culture barrel equipment (patent publication No.: CN208857314U) was used, filled with 20-100 L of synthetic medium (as shown in Table 1) and ventilated with air at 0-1 m³/h, specifically: the medium was a synthetic medium + 0.2 mg/L-0.5 mg/L 6-BA (6-benzyladenine), 2 mg/L-4 mg/L NAA (naphthalene acetic acid) and 30 g/L saccharose, an inoculation amount was 1-20 g of dry cells per liter of medium, a temperature was 25 ± 2°C, an illumination amount was 1,000-3,000 lxu and an illumination time was 12-24 h. Cells were filtered and harvested after suspension culturing for 14 days.

The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 15% of the cells (dry weight), the content of the chlorogenic acid accounted for more than 1.03%, the content of the syringin accounted for more than 0.85%, the content of the 1,5-dicaffeoylquinic acid accounted for more than 1.6%, and the content of the rutin accounted for more than 0.06%.

### Content determination

(1) Measurement of dry cell weight
   Cell culture solution was poured into a 120-mesh stainless steel mesh and filtered to harvest cells, the cells were washed with distilled water 3 times, moisture on the surface of the cells was fully absorbed with absorbent paper, a fresh weight was measured, wet cells were placed in an oven at 60°C and baked to a constant weight to obtain a dry cell weight.
(2) Method for determining total flavonoids of *Saussurea involucrata*:
   Preparation of control solution: 10 mg of rutin control substance dried to a constant weight at 120°C was precisely weighed and placed in a 50 mL volumetric flask, an appropriate amount of methanol was added, the mixture was heated in water bath to dissolve the rutin control substance and cooled down, methanol was added to the graduation line, and the mixture was shaken well. Then a control solution was obtained (0.2 mg of anhydrous rutin was contained in 1 mL of control solution);
   Preparation of a standard curve: 1 mL, 2 mL, 3 mL, 4 mL, 5 mL and 6 mL of control solutions were precisely weighed and placed in 25 mL volumetric flasks respectively, water was added to 6 mL in each volumetric flask, 1 mL of 5% sodium nitrite solution was added, and the flasks were shaken well and placed for 6 min; 1 mL of 10% aluminum nitrate was added, and the flasks were shaken well and placed for 6 min; 10 mL of sodium hydroxide test solution was added, water was added to the graduation line, and the flasks were shaken well and placed for 15 min; a corresponding reagent was used as a blank, an absorbance was determined at 500 nm, and a standard curve was plotted with the absorbance as a longitudinal coordinate and a concentration as a horizontal coordinate;
   Determination: About 0.10 g of coarse powder of this product was taken, precisely weighed and placed in a Soxhlet extractor, about 90 mL of methanol was added, the mixture was heated to reflux until an extracting solution became colorless (the number of reflux times for each sample should not be less than 4 and 2 hours) and transferred into a 50 mL volumetric flask, methanol was added to the graduation line, and the flask was shaken well. 5 mL of the mixture was precisely weighed and placed in a 25 mL volumetric flask, an absorbance was determined from the step of "water was added to 6 mL" according to the method under preparation of a standard curve, and the weight (in mg) of the anhydrous rutin contained in the test solution was read out from the standard curve, and calculation was performed to obtain the result.
(3) Content determination of phenylpropanoids
   Cultured *Saussurea involucrata* cells dried to a constant weight were taken and ground to fine powder; about 1.000 mg of fine powder was precisely weighed, placed in a test tube with a stopper and extracted ultrasonically with 50 mL of 20% methanol for 1 h; a weight loss was supplemented with methanol, the mixture was centrifuged at 15,000 r/min for 20 min, a supernatant was taken and filtered through a 0.45 µm filter membrane, and the contents of rutin, chlorogenic acid, syringin and 1,5-dicaffeoylquinic acid were determined simultaneously through HPLC. Shiseido capcell pak C18 MG II chromatographic column (4.6 mm×250 mm, 5 µm);

A mobile phase A was 0.2% of phosphate buffered saline (containing 50 mmo/L KH₂PO₄), a mobile phase B was acetonitrile, and gradient elution was performed. Gradient conditions: 0 min (VA: VB=92: 8)-20 min (VA:VB=90:10)-50 min (VA: VB=70: 30)-60 min (VA:VB=50:50); flow velocity 1.0 mL/min⁻¹; column temperature 30°C; detection wavelength 265 nm (syringin); sample size 20 µL.

### Example 8 Addition of precursor cinnamic acid to the medium for mass production of Saussurea involucrata cultures rich in medicinal components

The cell line in example 4 was selected as seeds and transferred into a synthetic medium containing cinnamic acid, and a specific formula was as follows: the synthetic medium (as shown in Table 1) + cinnamic acid of a different concentration + 0.2 mg/L-0.5 mg/L 6-BA (6-benzyladenine), 2 mg/L-4 mg/L NAA (naphthalene acetic acid) and 30 g/L saccharose. The seeds were cultured with a shaker for more than 3 rounds, plant cell culture barrel equipment (patent publication No. CN208857314U) was adopted for seeds propagated and stably subcultured, and 20-100 L of medium was contained in the plant cell culture barrel equipment; an inoculation amount was 10 g/L (fresh weight), a culturing time was 14 days, an illumination intensity was around 1,500 lux, and an illumination time was 12 h/day.

After the culturing was completed, the culture solution was filtered, cells in an upper layer were collected, and a yield of dry products dried at 50°C for 24 h was determined to be 15 g/L.

The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 22% of the cells (dry weight), the content of the chlorogenic acid accounted for more than 1.66%, the content of the syringin accounted for more than 1.45%, the content of the 1,5-dicaffeoylquinic acid accounted for more than 2.81%, and the content of the rutin accounted for more than 0.066%.

### Example 9 Addition of precursor phenylalanine to the medium for mass production of Saussurea involucrata cultures rich in medicinal components

The cell line in example 4 was selected as seeds and transferred into a synthetic medium containing phenylalanine, and a specific formula was as follows: the synthetic medium (as shown in Table 1) + phenylalanine of a different concentration + 0.2 mg/L-0.5 mg/L 6-BA (6-benzyladenine), 2 mg/L-4 mg/L NAA (naphthalene acetic acid) and 30 g/L saccharose. The specific formula is shown in the Table. The seeds were cultured with a shaker for more than 3 rounds, seeds propagated and stably subcultured were transferred into a production culture device, the equipment in publication No. CN208857314U was used as the culture device, 10-50 L of medium was contained in the device, and the device was ventilated with air at 0-1 m³/h; an inoculation amount was 40 g/L (fresh weight), a culturing time was 15 days, an illumination intensity was around 1,500 lux, and an illumination time was 12 h/day.

After the culturing was completed, the culture solution was filtered, cells in an upper layer were collected, and a yield of dry products dried at 50°C for 24 h was determined to be 15 g/L.

The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 22.7% of the cells (dry weight), the content of the chlorogenic acid accounted for more than 1.28%, the content of the syringin accounted for more than 2.07%, the content of the 1,5-dicaffeoylquinic acid accounted for more than 2.48%, and the content of the rutin accounted for more than 0.078%.

### Example 10 Addition of sodium acetate to the medium for mass production of Saussurea involucrata cultures rich in medicinal components

The cell line in example 4 was selected as seeds and transferred into a synthetic medium containing sodium acetate, and a specific formula was as follows: the synthetic medium (as shown in Table 1) + sodium acetate of a different concentration + 0.2 mg/L-0.5 mg/L 6-BA (6-benzyladenine), 2 mg/L-4 mg/L NAA (naphthalene acetic acid) and 30 g/L saccharose. The specific formula is shown in the table. The seeds were cultured with a shaker for more than 3 rounds, seeds propagated and stably subcultured were transferred into a production culture device, the culture device was the equipment in CN208857314U, 10-50 L of medium was contained in the device, and the device was ventilated with air at 0-1 m³/h; an inoculation amount was 50 g/L (fresh weight), a culture temperature was 25 ±2°C, a culturing time was 12 days, an illumination intensity was around 1,500 lux, and an illumination time was 12 h/day.

After the culturing was completed, the culture solution was filtered, cells in an upper layer were collected, and a yield of dry products dried at 50°C for 24 h was determined to be 15 g/L.

The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 21.8% of the cells (dry weight), the content of the chlorogenic acid accounted for more than 1.79%, the content of the syringin accounted for more than 1.22%, the content of the 1,5-dicaffeoylquinic acid accounted for more than 2.35%, and the content of the rutin accounted for more than 0.064%.

### Example 11 Addition of tyrosine to the medium in the middle stage of culturing for mass production of Saussurea involucrata cultures rich in medicinal components

Preparation of medium:
The culture solution was prepared by adding 0.4 mg/L 6-BA (6-benzyladenine), 2 mg/L NAA (naphthalene acetic acid) and 30 g/L saccharose to a synthetic medium (as shown in Table 1), and the pH value was 6.0; high temperature sterilization: 121 °C, 20 min.

Preparation of inducer:
0.04g of tyrosine was weighed, dissolved in 100 mL of pure water, and sterilized at 121 °C for 20 min for later use

Production of cultures:
The cell line in example 4 was selected as a seed and transferred into a culture flask for culturing and propagation. A shaker was used for culturing, seeds were cultured for more than 3 rounds, seeds propagated and stably subcultured were transferred into a production culture device, the culture device was the culture barrel in patent No. CN208857315U, 40 L of medium was contained in the device, and the device was ventilated with air at 0-1 m³/h; an inoculation amount was 30 g/L (fresh weight), a culture temperature was 25 ± 2 °C, an illumination intensity was 1,000-2,000 lux, and an illumination time was 12 h/day. On the 10^{th} day of culturing, 10-200 ml of inducer was added to a culture bag, and cultures were collected after 6 days of culturing.

Collection of cultures:
After the culturing was completed, the culture solution was filtered, cells in an upper layer were collected and oven-dried at 50°C for 24 h, and a weight of cultures after being dried was determined to be 18.2 g/L.

Content determination:
The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 19.4% of the cells (dry weight), the content of the chlorogenic acid accounted for more than 0.62%, the content of the syringin accounted for more than 1.49%, the content of the 1,5-dicaffeoylquinic acid accounted for more than 2.75%, and the content of the rutin accounted for more than 0.062%.

### Example 12 Addition of oxalic acid to the medium in the middle stage of culturing for mass production of Saussurea involucrata cultures rich in medicinal components

Preparation of medium:
The culture solution was prepared by adding 0.4 mg/L 6-BA (6-benzyladenine), 2 mg/L NAA (naphthalene acetic acid) and 30 g/L saccharose to a synthetic medium (as shown in Table 1), and the pH value was 6.0; high temperature sterilization: 121 °C, 20 min.

Preparation of inducer:
0.45g of oxalic acid was weighed, dissolved in 1,000 mL of pure water and prepared into 5 mmol/L inducer, a pH value was adjusted to 5.8 with NaOH, and the inducer was sterilized at 121 °C for 20 min for later use

Production of cultures:
The cell line in example 4 was selected as seeds and transferred into a culture flask for culturing and propagation. A shaker was used for culturing, seeds were cultured for more than 3 rounds, seeds propagated and stably subcultured were transferred into a production culture device, the culture device was the culture equipment in patent publication No. CN208857314U, 16 L of medium was contained in the device, and the device was ventilated with air at 0-1 m³/h. The medium was a synthetic medium (as shown in the table) + 0.2 mg/L-0.5 mg/L 6-BA (6-benzyladenine), 2 mg/L-4 mg/L NAA (naphthalene acetic acid) and 30 g/L saccharose. An inoculation amount was 30 g/L (fresh weight), a culture temperature was 25 ±2°C, an illumination intensity was 1,000-2,000 lxu and an illumination time was 12 h/day. On the 10^{th} day of culturing, the inducer was added to a culture barrel according to the proportion of 0.2-4 ml/L medium, and cultures were collected after 6 days of culturing.

Collection of cultures:
After the culturing was completed, the culture solution was filtered, cells in an upper layer were collected and oven-dried at 50°C for 24 h, and a weight of cultures after being dried was determined to be 16 g/L.

Content determination:
The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 19.6% of the cells (dry weight), the content of the chlorogenic acid accounted for more than 1.27%, the content of the syringin accounted for more than 0.95%, the content of the 1,5-dicaffeoylquinic acid accounted for more than 1.72%, and the content of the rutin accounted for more than 0.071%.

### Example 13 Addition of inducer methyl jasmonate to the medium in the middle stage to improve phenylpropanoid compounds in Saussurea involucrata cultures

Preparation of medium:
The culture solution was prepared by adding 0.4 mg/L 6-BA (6-benzyladenine), 2 mg/L NAA (naphthalene acetic acid) and 30 g/L saccharose to a dedicated medium (as shown in Table 1), and the pH value was 6.0; high temperature sterilization: 121 °C, 20 min.

Preparation of inducer:
0.5 g of methyl jasmonate was weighed, dissolved in 100 mL of pure water, and sterilized at 121 °C for 20 min for later use

Production of cultures:
The cell line in example 4 was selected as seeds and transferred into a culture flask for culturing and propagation. A shaker was used for culturing, seeds were cultured for more than 3 rounds, seeds propagated and stably subcultured were transferred into a production culture device, the culture device was the culture equipment in patent publication No. CN208857314U, 10-50 L of medium was contained in the device, and the device was ventilated with air at 0-1 m³/h. The medium was a synthetic medium (as shown in Table 1) + 0.2 mg/L-0.5 mg/L 6-BA (6-benzyladenine), 2 mg/L-4 mg/L NAA (naphthalene acetic acid) and 30 g/L saccharose. An inoculation amount was 30 g/L (fresh weight), a culture temperature was 25±2°C, an illumination intensity was 1000-2000 lxu and an illumination time was 12 h/day. On the 10^{th} day of culturing, the inducer was added to a culture barrel according to the proportion of 0.2-6 ml/L medium, and cultures were collected after 6 days of culturing.

Collection of cultures:
After the culturing was completed, the culture solution was filtered, cells in an upper layer were collected and oven-dried at 50°C for 24 h, and a weight of cultures after being dried was determined to be 20.4 g/L.

Content determination:
The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoid accounted for more than 24.1% of the cells (dry weight), the content of the chlorogenic acid accounted for more than 2.44%, the content of the syringin accounted for more than 1.89%, the content of the 1,5-dicaffeoylquinic acid accounted for more than 4.12%, and the content of the rutin accounted for more than 0.081%.

### Example 14 Addition of inducer silver nitrate to the medium in the middle stage to improve phenylpropanoid compounds in Saussurea involucrata cultures

Preparation of medium:
The culture solution was prepared by adding 0.4 mg/L 6-BA (6-benzyladenine), 2 mg/L NAA (naphthalene acetic acid) and 30 g/L saccharose to a synthetic medium (as shown in Table 1), and the pH value was 6.0; high temperature sterilization: 121 °C, 20 min.

Preparation of inducer:
0.5 g of silver nitrate was weighed and dissolved in 100 mL of pure water, the solution was poured into a black culture flask and sterilized at 121 °C for 20 min for later use;

Production of cultures:
The cell line in example 4 was selected as seeds and transferred into a culture flask for culturing and propagation. A shaker was used for culturing, seeds were cultured for more than 3 rounds, seeds propagated and stably subcultured were transferred into a production culture device, the culture device was the culture equipment in patent publication No. CN208857314U, 10-50 L of medium was contained in the device, and the device was ventilated with air at 0-1 m³/h. The medium was a synthetic medium (as shown in Table 1) + 0.2 mg/L-0.5 mg/L 6-BA (6-benzyladenine), 2 mg/L-4 mg/L NAA (naphthalene acetic acid) and 30 g/L saccharose. An inoculation amount was 30 g/L fresh weight, a culture temperature was 25 ± 2°C, an illumination intensity was 1,000-2,000 lxu and an illumination time was 12 h/day. On the 10^{th} day of culturing, the inducer was added to a culture barrel according to the proportion of 0.2-5 ml/L medium, and cultures were collected after 6 days of culturing.

Collection of cultures:
After the culturing was completed, the culture solution was filtered, cells in an upper layer were collected and oven-dried at 50°C for 24 h, and a weight of cultures after being dried was determined to be 16 g/L.

Content determination:
The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 19.5% of the cells (dry weight), the content of the chlorogenic acid accounted for more than 1.47%, the content of the syringin accounted for more than 1.38%, the content of the 1,5-dicaffeoylquinic acid accounted for more than 2.97%, and the content of the rutin accounted for more than 0.065%.

### Example 15 Addition of inducer salicylic acid to the medium in the middle stage to improve phenylpropanoid compounds in Saussurea involucrata cultures

Preparation of medium:
   The culture solution was prepared by adding 0.4 mg/L 6-BA (6-benzyladenine), 2 mg/L NAA (naphthalene acetic acid) and 30 g/L saccharose to a dedicated medium (as shown in Table 1), and the pH value was 6.0; high temperature sterilization: 121 °C, 20 min;
Preparation of inducer:
   0.5 g of salicylic acid was weighed and dissolved in 100 mL of pure water, the solution was poured into a black culture flask and sterilized at 121 °C for 20 min for later use;
Production of cultures:
   The cell line in example 4 was selected as seeds and transferred into a culture flask for culturing and propagation. A shaker was used for culturing, seeds were cultured for more than 3 rounds, seeds propagated and stably subcultured were transferred into a production culture device, the culture device was the culture equipment in patent No. CN208577726U, 10-100 L of synthetic medium was contained in the device, and the device was ventilated with air at 0-1 m³/h. The medium was a synthetic medium (as shown in Table 1) + 0.2 mg/L-0.5 mg/L 6-BA (6-benzyladenine), 2 mg/L-4 mg/L NAA (naphthalene acetic acid) and 30 g/L saccharose. An inoculation amount was 30 g/L (fresh weight), a culture temperature was 25±2°C, an illumination intensity was 1,000-2,000 lxu and an illumination time was 12 h/day. On the 10^{th} day of culturing, the inducer was added to a culture bag according to the proportion of 0.2-8 ml/L medium, and cultures were collected after 6 days of culturing;
Collection of cultures:
   After the culturing was completed, the culture solution was filtered, cells in an upper layer were collected and oven-dried at 50°C for 24 h, and a weight of cultures after being dried was determined to be 16.8 g/L.
Content determination:
   The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 21.2% of the cells (dry weight), the content of the chlorogenic acid accounted for more than 1.86%, the content of the syringin accounted for more than 1.09%, the content of the 1,5-dicaffeoylquinic acid accounted for more than 3.55%, and the content of the rutin accounted for more than 0.072%.

### Example 16 Addition of combined inducers to the medium in the middle stage to improve phenylpropanoid compounds in Saussurea involucrata cultures

Preparation of medium:
   The culture solution was prepared by adding 0.4 mg/L 6-BA (6-benzyladenine), 2 mg/L NAA (naphthalene acetic acid) and 30 g/L saccharose to an MS medium (as shown in Table 2), and the pH value was 6.0; high temperature sterilization: 121°C, 20 min.
Preparation of inducer:
   0.5 g of salicylic acid, 0.5 g of silver nitrate and 0.5 g of methyl jasmonate were weighed and dissolved in 100 mL of pure water, and the solution was poured into a black culture flask and sterilized at 121 °C for 20 min for later use;
Production of cultures:
   The cell line in example 4 was selected as seeds and transferred into a culture flask for culturing and propagation. A shaker was used for culturing, seeds were cultured for more than 3 rounds, seeds propagated and stably subcultured were transferred into a production culture device, the culture device was the culture equipment in patent No. CN208577726U, 300 L of synthetic medium was contained in the device, and the device was ventilated with air at 0-50 m³/h. The medium was a synthetic medium (as shown in Table 1) + 0.2 mg/L-0.5 mg/L 6-BA (6-benzyladenine), 2 mg/L-4 mg/L NAA (naphthalene acetic acid) and 30 g/L saccharose. An inoculation amount was 30 g/L (fresh weight), a culture temperature was 25 ± 2°C, an illumination intensity was 1000-2000 lxu and an illumination time was 12 h/day. On the 10^{th} day of culturing, the inducer was added to a culture tank according to the proportion of 0.2-5 ml/L medium, and cultures were collected after 6 days of culturing.
Collection of cultures:
   After the culturing was completed, the culture solution was filtered, cells in an upper layer were collected and oven-dried at 50°C for 24 h, and a weight of cultures after being dried was determined to be 16 g/L.
Content determination:
   The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 18.1% of the cells (dry weight), the content of the chlorogenic acid accounted for more than 0.68%, the content of the syringin accounted for more than 0.77%, the content of the 1,5-dicaffeoylquinic acid accounted for more than 1.85%, and the content of the rutin accounted for more than 0.028%.

### Example 17 Addition of combined inducers to the medium in the middle stage to improve phenylpropanoid compounds in Saussurea involucrata cultures

Preparation of medium:
The culture solution was prepared by adding 0.4 mg/L 6-BA (6-benzyladenine), 2 mg/L NAA (naphthalene acetic acid) and 30 g/L saccharose to a dedicated medium (as shown in Table 1), and the pH value was 6.0; high temperature sterilization: 121 °C, 20 min.

Preparation of inducer:
10.0 g of salicylic acid and 10.0 g of silver nitrate were weighed and dissolved in 1,000 mL of pure water, and the solution was poured into a black culture flask and sterilized at 121 °C for 20 min for later use.

Production of cultures:
The shear-resistant cell line in example 6 was selected as seeds and transferred into a culture flask for culturing and propagation. A shaker was used for culturing, seeds were cultured for more than 3 rounds, seeds propagated and stably subcultured were transferred into a production culture device, the culture device was the culture equipment in patent No. CN208577726U, 300 L of synthetic medium was contained in the device, and the device was ventilated with air at 0-50 m³/h. The medium was a synthetic medium (as shown in Table 1) + 0.2 mg/L-0.5 mg/L 6-BA (6-benzyladenine), 2 mg/L-4 mg/L NAA (naphthalene acetic acid) and 30 g/L saccharose. An inoculation amount was 30 g/L fresh weight, a culture temperature was 25 ± 2°C, an illumination intensity was 1000-2000 lxu and an illumination time was 12 h/day. On the 10^{th} day of culturing, the inducer was added to a culture tank according to the proportion of 0.2-4 ml/L medium, and cultures were collected after 6 days of culturing.

Collection of cultures:
After the culturing was completed, the culture solution was filtered, cells in an upper layer were collected and oven-dried at 50°C for 24 h, and a weight of cultures after being dried was determined to be 16.9 g/L.

Content determination:
The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 18.27% of the cells (dry weight), the content of the chlorogenic acid accounted for more than 1.41%, the content of the syringin accounted for more than 1.04%, the content of the 1,5-dicaffeoylquinic acid accounted for more than 2.57%, and the content of the rutin accounted for more than 0.066%.

**Example 18** Addition of combined inducers to the medium in the middle stage to improve phenylpropanoid compounds in *Saussurea involucrata* cultures

Preparation of medium:
The culture solution was prepared by adding 0.4 mg/L 6-BA (6-benzyladenine), 2 mg/L NAA (naphthalene acetic acid) and 30 g/L saccharose to a dedicated medium (as shown in Table 1), and the pH value was 6.0; high temperature sterilization: 121 °C, 20 min.

Preparation of inducer:
10.0 g of methyl jasmonate, 10.0 g of salicylic acid and 10.0 g of silver nitrate were weighed and dissolved in 1,000 mL of pure water, and the solution was poured into a black culture flask and sterilized at 121 °C for 20 min for later use.

Production of cultures:
The shear-resistant cell line in example 6 was selected as seeds and transferred into a culture flask for culturing and propagation. A shaker was used for culturing, seeds were cultured for more than 3 rounds, seeds propagated and stably subcultured were transferred into a production culture device, the culture device was the culture equipment in patent No. CN208577726U, 300 L of synthetic medium was contained in the device, and the device was ventilated with air at 0-50 m³/h. The medium was a synthetic medium (as shown in Table 1)+ 0.2 mg/L-0.5 mg/L 6-BA (6-benzyladenine), 2 mg/L-4 mg/L NAA (naphthalene acetic acid) and 30 g/L saccharose. An inoculation amount was 30 g/L (fresh weight), a culture temperature was 25±2°C, an illumination intensity was 1,000-2,000 lxu and an illumination time was 12 h/day. On the 10^{th} day of culturing, the inducer was added to a culture tank according to the proportion of 0.2-6 ml/L medium, and cultures were collected after 6 days of culturing.

Collection of cultures:
After the culturing was completed, the culture solution was filtered, cells in an upper layer were collected and oven-dried at 50°C for 24 h, and a weight of cultures after being dried was determined to be 16.8 g/L.

Content determination:
The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 24.9% of the cells (dry weight), the content of the chlorogenic acid accounted for more than 2.58%, the content of the syringin accounted for more than 2.07%, the content of the 1,5-dicaffeoylquinic acid accounted for more than 4.37%, and the content of the rutin accounted for more than 0.095%.

### Example 19 Addition of combined inducers to the medium in the middle stage to improve phenylpropanoid compounds in Saussurea involucrata cultures

Preparation of medium:
The culture solution was prepared by adding 0.4 mg/L 6-BA (6-benzyladenine), 2 mg/L NAA (naphthalene acetic acid) and 30 g/L saccharose to a dedicated medium (as shown in Table 1), and the pH value was 6.0; high temperature sterilization: 121 °C, 20 min.

Preparation of inducer:
10.0 g of salicylic acid, 10.0 g of silver nitrate, 10.0 g of methyl jasmonate and 10.0 g of sodium nitroprusside were weighed and dissolved in 1000 mL of pure water, and the solution was poured into a black culture flask and sterilized at 121 °C for 20 min for later use.

Production of cultures:
The shear-resistant cell line in example 6 was selected as seeds and transferred into a culture flask for culturing and propagation. A shaker was used for culturing, seeds were cultured for more than 3 rounds, seeds propagated and stably subcultured were transferred into a production culture device, the culture device was the culture equipment in patent No. CN208577726U, 300 L of synthetic medium was contained in the device, and the device was ventilated with air at 0-50 m³/h. The medium was a synthetic medium (as shown in Table 1) + 0.2 mg/L-0.5 mg/L 6-BA (6-benzyladenine), 2 mg/L-4 mg/L NAA (naphthalene acetic acid) and 30 g/L saccharose. An inoculation amount was 30 g/L fresh weight, a culture temperature was 25±2°C, an illumination intensity was 1,000-2,000 lxu and an illumination time was 12 h/day. On the 10^{th} day of culturing, the inducer was added to a culture tank according to the proportion of 0.2-6 ml/L medium, and cultures were collected after 6 days of culturing.

Collection of cultures: After the culturing was completed, the culture solution was filtered, cells in an upper layer were collected and oven-dried at 50°C for 24 h, and a weight of cultures after being dried was determined to be 16.3 g/L.

Content determination: The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 23.5% of the cells (dry weight), the content of the chlorogenic acid accounted for more than 2.27%, the content of the syringin accounted for more than 1.33%, the content of the 1,5-dicaffeoylquinic acid accounted for more than 3.64%, and the content of the rutin accounted for more than 0.067%.

### Example 20 Addition of bypass metabolism inhibitor to the medium in the late stage to improve phenylpropanoid compounds in Saussurea involucrata cultures

Preparation of medium:
The culture solution was prepared by adding 0.4 mg/L 6-BA (6-benzyladenine), 2 mg/L NAA (naphthalene acetic acid) and 30 g/L saccharose to a dedicated medium (as shown in Table 1), and the pH value was 6.0; high temperature sterilization: 121 °C, 20 min.

Preparation of inhibitor:
10.0 g of arginine, 10.0 g of D-alanine and 15.0 g of sodium citrate were weighed and dissolved in 1000 mL of pure water, and the solution was poured into a black culture flask and sterilized at 121 °C for 20 min for later use.

Production of cultures:
The cell line in example 4 was selected as seeds and transferred into a culture flask for culturing and propagation. A shaker was used for culturing, seeds were cultured for more than 3 rounds, seeds propagated and stably subcultured were transferred into a production culture device, the culture device was the culture equipment in patent publication No. CN208857314U, 10-50 L of medium was contained in the device, and the device was ventilated with air at 0-1 m³/h. The medium was a synthetic medium (as shown in Table 1)+ 0.2 mg/L-0.5 mg/L 6-BA (6-benzyladenine), 2 mg/L-4 mg/L NAA (naphthalene acetic acid) and 30 g/L saccharose. An inoculation amount was 30 g/L fresh weight, a culture temperature was 25 ± 2°C, an illumination intensity was 1,000-2,000 lxu and an illumination time was 12 h/day. On the 13^{th} day of culturing, the metabolism inhibitor was added to a culture barrel according to the proportion of 0.2-5 ml/L medium, and cultures were collected after 2 days of continued culturing.

Collection of cultures:
After the culturing was completed, the culture solution was filtered, cells in an upper layer were collected and oven-dried at 50°C for 24 h, and a weight of cultures after being dried was determined to be 14.8 g/L.

Content determination:
The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 18.5% of the cells (dry weight), the content of the chlorogenic acid accounted for more than 1.87%, the content of the syringin accounted for more than 1.1%, the content of the 1,5-dicaffeoylquinic acid accounted for more than 2.3%, and the content of the rutin accounted for more than 0.042%.

### Example 21 Addition of bypass metabolism inhibitor to the medium in the late stage to improve phenylpropanoid compounds in Saussurea involucrata cultures

Preparation of medium:
The culture solution was prepared by adding 0.4 mg/L 6-BA (6-benzyladenine), 2 mg/L NAA (naphthalene acetic acid) and 30 g/L saccharose to a dedicated medium (as shown in Table 1), and the pH value was 6.0; high temperature sterilization: 121 °C, 20 min.

Preparation of inhibitor:
10.0 g of arginine, 10.0 g of D-alanine, 5.0 g of 5-MT and 15.0 g of sodium citrate were weighed and dissolved in 1000 mL of pure water, and the solution was poured into a black culture flask and sterilized at 121 °C for 20 min for later use.

Production of cultures:
The cell line in example 4 was selected as seeds and transferred into a culture flask for culturing and propagation. A shaker was used for culturing, seeds were cultured for more than 3 rounds, seeds propagated and stably subcultured were transferred into a production culture device, the culture device was the culture equipment in patent publication No. CN208857314U, 10-50 L of medium was contained in the device, and the device was ventilated with air at 0-1 m³/h. The medium was a synthetic medium (as shown in Table 1) + 0.2 mg/L-0.5 mg/L 6-BA (6-benzyladenine), 2 mg/L-4 mg/L NAA (naphthalene acetic acid) and 30 g/L saccharose. An inoculation amount was 30 g/L fresh weight, a culture temperature was 25±2°C, an illumination intensity was 1000-2000 lxu and an illumination time was 12 h/day. On the 13^{th} day of culturing, the metabolism inhibitor was added to a culture barrel according to the proportion of 0.2-5 ml/L medium, and cultures were collected after 2 days of continued culturing.

Collection of cultures:
After the culturing was completed, the culture solution was filtered, cells in an upper layer were collected and oven-dried at 50°C for 24h, and a weight of cultures after being dried was determined to be 15.0 g/L.

Content determination:
The content of total flavonoids in the cells was measured with a spectrophotometer, and the contents of synthetic secondary metabolites (i.e., rutin, chlorogenic acid, syringin and 1,5-dicaffeoylquinic acid) of *Saussurea involucrata* were detected through HPLC. The results showed that the content of the flavonoids accounted for more than 20% of the cells (dry weight), the content of the chlorogenic acid accounted for more than 2.0%, the content of the syringin accounted for more than 1.2%, the content of the 1,5-dicaffeoylquinic acid accounted for more than 2.2%, and the content of the rutin accounted for more than 0.052%.

**Table 1 Synthetic Medium Formula**

| **Composition of Medium** | | | | | |
|---|---|---|---|---|---|
| **Chemical Component** | **mg/L** | **Chemical Component** | **mg/L** | **Chemical Component** | **mg/L** |
| Potassium nitrate | 3000 | Manganese sulfate tetrahydrate | 22.3 | Ferrous sulfate heptahydrate | 27.8 |
| Ammonium sulfate | 793 | Zinc sulfate heptahydrate | 8.6 | Inositol | 100 |
| Diammonium phosphate | 340 | Sodium molybdate dihydrate | 0.25 | Thiamine hydrochloride VB1 | 0.1 |
| Magnesium sulfate heptahydrate | 74 | Copper sulfate pentahydrate | 0.025 | Pyridoxine hydrochloride VB6 | 0.5 |
| Calcium chloride dihydrate | 88 | Cobalt chloride hexahydrate | 0.025 | Nicotinic acid VB₅ or VPP | 0.5 |
| Potassium iodide | 0.83 | Ethylene diamine tetraacetic acid disodium | 37.3 | Glycine | 2 |
| Boric acid | 2 | | | | |

**Table 2 MS Medium Formula**

| **Composition of Medium** | | | | | |
|---|---|---|---|---|---|
| **Chemical Component** | **mg/L** | **Chemical Component** | **mg/L** | **Chemical Component** | **mg/L** |
| Potassium nitrate | 1900 | Manganese sulfate tetrahydrate | 22.3 | Ferrous sulfate heptahydrate | 27.8 |
| Ammonium nitrate | 1650 | Zinc sulfate heptahydrate | 8.6 | Inositol | 100 |
| Potassium dihydrogen phosphate | 340 | Sodium molybdate dihydrate | 0.25 | Thiamine hydrochloride VB1 | 0.1 |
| Magnesium sulfate heptahydrate | 370 | Copper sulfate pentahydrate | 0.025 | Pyridoxine hydrochloride VB6 | 0.5 |
| Calcium chloride dihydrate | 440 | Cobalt chloride hexahydrate | 0.025 | Nicotinic acid VB5 or VPP | 0.5 |
| Potassium iodide | 0.83 | Ethylene diamine tetraacetic acid disodium | 37.3 | Glycine | 2 |
| Boric acid | 6.2 | | | | |

**Table 3 1/2MS Medium Formula**

| **Composition of Medium** | | | | | |
|---|---|---|---|---|---|
| **Chemical Component** | **mg/L** | **Chemical Component** | **mg/L** | **Chemical Component** | **mg/L** |
| Potassium nitrate | 950 | Manganese sulfate tetrahydrate | 22.3 | Ferrous sulfate heptahydrate | 27.8 |
| Ammonium nitrate | 825 | Zinc sulfate heptahydrate | 8.6 | Inositol | 100 |
| Potassium dihydrogen phosphate | 170 | Sodium molybdate dihydrate | 0.25 | Thiamine hydrochloride VB1 | 0.1 |
| Magnesium sulfate heptahydrate | 185 | Copper sulfate pentahydrate | 0.025 | Pyridoxine hydrochloride VB6 | 0.5 |
| Calcium chloride dihydrate | 220 | Cobalt chloride hexahydrate | 0.025 | Nicotinic acid VB5 or VPP | 0.5 |
| Potassium iodide | 0.83 | Ethylene diamine tetraacetic acid disodium | 37.3 | Glycine | 2 |
| Boric acid | 6.2 | | | | |

For any of those skilled in the art, many possible alterations and modifications can be made to the technical solution of the present invention by using the technical contents disclosed above, or the technical solution can be amended into equivalent embodiments with equivalent changes without departing from the scope of the technical solution of the present invention. Therefore, any simple amendments, equivalent changes and modifications made to the above-mentioned embodiments according to the technical essence of the present invention without departing from the contents of the technical solution of the present invention shall fall within the protection scope of the technical solution of the present invention.

## Claims

1. A medium for improving a content of flavonoid phenylpropanoid compounds in a *Saussurea involucrata* cell culture, wherein in the medium, a phosphorus concentration is 0.5-3 mmol/L, a NO₃⁻ ion concentration is 20-35 mmol/L, a NH₄⁺ ion concentration is 0-30 mmol/L, a Ca²⁺ ion concentration is 0.5-3 mmol/L, a Mg²⁺ ion concentration is 0.2-1.5 mmol/L, and a boron ion concentration is 0.02-0.1 mmol/L.

2. The medium for improving a content of flavonoid phenylpropanoid compounds in a *Saussurea involucrata* cell culture of claim 1, wherein in the medium, a phosphorus concentration is 2.7 mmol/L, a NO₃⁻ ion concentration is 30 mmol/L, a NH₄⁺ ion concentration is 18 mmol/L, a Ca²⁺ ion concentration is 0.6 mmol/L, a Mg²⁺ ion concentration is 0.3 mmol/L, and a boron ion concentration is 0.03 mmol/L.

3. The medium for improving a content of flavonoid phenylpropanoid compounds in a *Saussurea involucrata* cell culture of claim 1, wherein the medium comprises potassium nitrate 3000 mg/L, ammonium sulfate 793 mg/L, diammonium hydrogen phosphate 340 mg/L, magnesium sulfate heptahydrate 74 mg/L, calcium chloride dihydrate 88 mg/L, potassium iodide 0.83 mg/L, boric acid 2 mg/L, manganese sulfate tetrahydrate 22.3 mg/L, ferrous sulfate heptahydrate 27.8 mg/L, zinc sulfate heptahydrate 8.6 mg/L, inositol 100 mg/L, sodium molybdate dihydrate 0.25 mg/L, thiamine hydrochloride 0.1 mg/L, copper sulfate pentahydrate 0.025 mg/L, pyridoxine hydrochloride 0.5 mg/L, cobalt chloride hexahydrate 0.025 mg/L, nicotinic acid 0.5 mg/L, ethylene diamine tetraacetic acid disodium 37.3 mg/L, glycine 2 mg/L.

4. An inducer for improving a content of flavonoid phenylpropanoid compounds in a *Saussurea involucrata* cell culture, wherein the inducer is one or more of SNP inducer, MeJ inducer, SA inducer, and silver ion and silver ion complex inducer; wherein a concentration of the SNP inducer added is 1-50 mmol/L, a concentration of the MeJ inducer added is 1-50 mg/L, a concentration of the SA inducer added is 1-50 mg/L, and a concentration of the silver ion and silver ion complex inducer added is 1-50 mg/L; preferably, the concentration of the SNP inducer added is 10 mmol/L, the concentration of the MeJ inducer added is 20 mg/L, the concentration of the SA inducer added is 30 mg/L, and the concentration of the silver ion and silver ion complex inducer added is 20 mg/L.

5. A precursor for improving a content of flavonoid phenylpropanoid compounds in a *Saussurea involucrata* cell culture, wherein the precursor is one or more of cinnamic acid, phenylalanine, sodium acetate, tyrosine and oxalic acid; wherein a concentration of the cinnamic acid added is 1-20 mg/L, a concentration of the phenylalanine added is 1-20 mg/L, a concentration of the sodium acetate added is 1-20 mg/L, a concentration of the tyrosine added is 1-50 mg/L, and a concentration of the oxalic acid added is 1-20 mg/L; preferably, the concentration of the cinnamic acid added is 10 mg/L, the concentration of the phenylalanine added is 10 mg/L, the concentration of the sodium acetate added is 10 mg/L, the concentration of the tyrosine added is 20 mg/L, and the concentration of the oxalic acid added is 10 mg/L.

6. A bypass metabolism inhibitor for improving a content of flavonoid phenylpropanoid compounds in a *Saussurea involucrata* cell culture, wherein the bypass metabolism inhibitor is one or more of arginine, trisodium citrate, sodium fluoride, cyclohexylalanine and 5-MT; wherein a concentration of the arginine added is 0-100 mg/L, a concentration of the trisodium citrate added is 0-50 mg/L, a concentration of the sodium fluoride added is 0-100 mg/L, a concentration of the cyclohexylalanine added is 0-100 mg/L, and a concentration of the 5-MT added is 0-100 mg/L.

7. The bypass metabolism inhibitor for improving a content of flavonoid phenylpropanoid compounds in a *Saussurea involucrata* cell culture of claim 6, wherein the concentration of the arginine added is 10 mg/L, the concentration of the trisodium citrate added is 15 mg/L, the concentration of the sodium fluoride added is 15 mg/L, the concentration of the cyclohexylalanine added is 10 mg/L, and the concentration of the 5-MT added is 10 mg/L.

8. A method for improving a content of flavonoid phenylpropanoid compounds in a *Saussurea involucrata* cell culture, wherein the method comprises the following steps:
(1) acquisition of a primitive cell line:
using a seed embryo of *Saussurea involucrata* or a plant germinated from a seed as an explant, inducing a callus with an induction medium, and performing repeated subculturing to obtain a *Saussurea involucrata* cell line;
(2) inoculating the *Saussurea involucrata* cell line obtained in step (1) into a medium for culturing, with an inoculation amount of 1-50 g of cells (fresh weight) per liter of medium, an illumination amount of 1,000-3,000 lxu, an illumination time of 12-24 h/day and a shaker speed of 100-130 rpm, performing subculturing one time every 7 days-20 days, and establishing a stable and high-yield *Saussurea involucrata* suspension cell culture line after 3-4 rounds of subculturing; and
(3) large-scale culturing: selecting the culture in step (2), inoculating the culture into a medium for culturing, with an inoculation amount of 10-80 g of cells (fresh weight) per liter of medium, and filtering and harvesting cells after suspension culturing for 14-20 days;
wherein the medium in step (2) and step (3) is the medium of any one of claims 1-3; or the medium is a medium comprising the precursor of claim 5; or the medium is a medium comprising the inducer of claim 4; or the medium is a medium comprising the bypass metabolism inhibitor of claim 6 or 7.

9. The method for improving a content of flavonoid phenylpropanoid compounds in a *Saussurea involucrata* cell culture of claim 8, wherein when the precursor is cinnamic acid, phenylalanine or sodium acetate, the precursor is added during inoculation; when the precursor is tyrosine or oxalic acid, the precursor is added in the middle stage of culturing; the inducer is added in the middle stage of culturing; and the bypass metabolism inhibitor is added in the late stage of culturing.

10. The method for improving a content of flavonoid phenylpropanoid compounds in a *Saussurea involucrata* cell culture of claim 8, wherein before the large-scale culturing in step (3), the cells are inoculated into culture flasks containing 5-20 glass beads with a diameter of 1-10 mm, the culturing conditions are the same as those of the primitive cells, the culture flasks with *Saussurea involucrata* plants not growing well are discarded in the subculturing process, and a shear-resistant, stable and high-yield *Saussurea involucrata* suspension cell culture line is established after 10 rounds.
